# EUROPÄISCHE PATENTSCHRIFT

(11) **EP 0 658 097 B1**
(45) Veröffentlichungstag und Bekanntmachung des Hinweises auf die Patenterteilung: **16.10.1996**
(21) Anmeldenummer: 93919120.1
(22) Anmeldetag: 20.08.1993
(51) Int. Cl.: A61K 7/32

(54) **DESODORIERENDE KOSMETISCHE MITTEL MIT EINEM GEHALT AN FETTSÄUREN**
DEODORIZING COSMETICS CONTAINING FATTY ACIDS
AGENT COSMETIQUE DEODORANT CONTENANT DES ACIDES GRAS

(30) Priorität: 05.09.1992 DE 4229737
(43) Veröffentlichungstag der Anmeldung: 21.06.1995
(73) Patentinhaber: Beiersdorf Aktiengesellschaft, D-20245 Hamburg (DE)
(72) Erfinder: KLIER, Manfred, D-20521 Aumühle (DE); RÖCKL, Manfred, D-22880 Wedel (DE); WOLF, Florian, D-20251 Hamburg (DE)
(86) Internationale Anmeldenummer: EP9302236
(87) Internationale Veröffentlichungsnummer: WO9405253

(56) Entgegenhaltungen:
- EP-A- 0 003 172
- EP-A- 0 036 134
- EP-A- 0 243 145
- DE-A- 2 532 693
- GB-A- 2 024 010
- US-A- 4 477 361
- JANISTYN 'Handbuch der Kosmetika und Riechstoffe, Band 1: Die kosmetischen Grundstoffe' 1980 , HUTHIG VERLAG , HEIDELBERG,DE siehe seite 366, Absatz Kokosfett

## Beschreibung

Die vorliegende Erfindung betrifft kosmetische Desodorantien. Solche Formulierungen dienen dazu, Körpergeruch zu beseitigen, der entsteht, wenn der an sich geruchlose frische Schweiß durch Mikroorganismen zersetzt wird. Den handelsüblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Es wurde überraschend gefunden, und darin liegt die Lösung all dieser Aufgaben, daß kosmetische Desodorantien mit einem wirksamen Gehalt eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C₆₋₂₀,
(c) unter Verzicht auf Glycerylfettsäureester, ethoxylierte Glycerylfettsäureester und propoxylierte Glycerylfettsäureester
(d) im pH-Bereich unter 7 vorliegend,
den Nachteilen des Standes der Technik abhelfen.

Aus der EP-OS 0 243 145 ist zwar eine antimikrobiell wirksame topische pharmazeutische Zusammensetzung bekannt, die laut den unabhängigen Ansprüchen 2 und 3 eine ternäre Mischung aus einem Glycerylfettsäureester, und/oder einem ethoxylierten Glycerylfettsäureester und/oder einem propoxylierten Glycerylfettsäureester, einer Mischung aus Fettsäuren und einem pharmazeutisch annehmbaren Träger bekannt, wobei die Fettsäuren aus einer ersten Fettsäure einer Kettenlänge von C₆₋₁₈ und einer zweiten Fettsäure einer Kettenlänge von C₆₋₁₈ bestehen.

Die Dodecansäure und andere erfindungsgemäße Fettsäuren werden zwar formal von diesen generischen Fettsäureformeln umfaßt. Die in dieser Schrift beschriebene antimikrobielle Wirkung wird jedoch allein der Wirkung der Glycerylfettsäureester, der ethoxylierten Glycerylfettsäureester und/oder der propoxylierten Glycerylfettsäureester zugeschrieben, welche zwingend allen dort beschriebenen Zusammensetzungen zugrunde liegen.

Diese Schrift weist in eine andere Richtung als die, die durch die vorliegende Erfindung gegeben wird. In überraschender Weise hat sich nämlich herausgestellt, daß ein Zusatz von Glycerylfettsäureestern, ethoxylierten Glycerylfettsäureestern und/oder propoxylierten Glycerylfettsäureestern die antimikrobielle Wirkung des erfindungsgemäßen Gemisches aus Laurinsäure und anderen Fettsäuren erheblich mindert, in manchen Fällen, bei bestimmten Konzentrationen sogar gänzlich aufhebt.

Diese Schrift offenbart nur Pharmaka und keine Kosmetika.

Ferner ist aus der EP-OS 0 465 423 eine pharmazeutische Zusammensetzung zur Bekämpfung von Mikroorganismen bekannt, welche im wesentlichen einen inerten Träger und einen aktiven Bestandteil umfaßt, welcher aus einer wirksamen Menge einer oder mehrerer Verbindungen, gewählt aus Fettsäuren einer Kettenlänge von C₄₋₁₄ und deren Monoglyceriden sowie der einfach oder mehrfach ungesättigten Fettsäuren einer Kettenlänge von C₁₄₋₂₂ und deren Monoglyceriden, besteht. Einen Hinweis in die Richtung der vorliegenden Erfindung findet sich in auch dieser Schritt nicht.

Diese Schrift offenbart gleichfalls nur Pharmaka und keine Kosmetika.

Schließlich ist bekannt, Seifen, also Salze der erfindungsgemäßen Säuren in Kombination einzusetzen. Diese sind jedoch nicht erfindungsgemäß aktiv. In überraschender Weise entfalten die erfindungsgemäßen Gemische ihre vorteilhafte antimikrobielle und desodorierende kosmetische Wirkung nur im pH-Bereich unter 7.

Die desodorierende Wirkung der erfindungsgemäßen Gemische beruht in erster Linie auf deren selektiver Toxizität für grampositive, insbesondere coryneforme Bakterien. Diese werden als die für die Zersetzung des apokrinen Schweißes hauptsächlich verantwortlichen Keime angesehen. Da diese Gemische zugleich völlig unschädlich für den Menschen und andere Warmblüter sind, sind sie ideal für die Verwendung in kosmetischen Desodorantien geeignet.

Insbesondere war überraschend, daß, obwohl Laurinsäure, die im allgemeinen den Hauptbestandteil der erfindungsgemäßen Gemische darstellt, als bei sehr empfindlichen Personen schwach hautreizend eingeschätzt wird, die erfindungsgemäßen Gemische aber nicht einmal bei Probanden mit empfindlicher Haut auch nur die geringsten Reizerscheinungen zeigten.

Ferner war überraschend, daß die erfindungsgemäßen Gemische in synergistischer Weise gegen coryneforme Bakterien wirken. In "Antiseptika", Teil 3, Untertitel ''Antibakterielle, antifungielle und antivirale Antiseptik - ausgewählte Wirkstoffe'', Gustav Fischer Verlag, Stuttgart, New York, 1987, Ss. 250 ff. wird dargestellt, daß zwar Laurinsäure gegen coryneforme Bakterien gut wirksam ist, die anderen C₆₋₂₀- Fettsäuren jedoch bestenfalls schwache Wirkung zeitigen. Am angegebenen Ort wird beschrieben, daß einige der erfindungsgemäß einzusetzenden Fettsäuren über gewisse mikrobizide Eigenschaffen verfügen, über ein synergistisches Zusammenspiel der einzelnen Fettsäurekomponenten miteinander war bisher nichts bekannt.

Bevorzugt werden die C₆₋₂₀-Fettsäuren gewählt aus der Gruppe Capronsäure (Hexansäure), Caprylsäure (Octansäure), Pelargonsäure (Nonansäure), Caprinsäure (Decansäure), Myristinsäure (Tetradecansäure), Palmitinsäure (Hexadecansäure) und Stearinsäure (Octadecansäure).

Es ist von Vorteil, folgende Gewichtsverhältnisse zu wählen, unabhängig, ob ein Gemisch der Laurinsäure mit einer oder mehreren weiteren Fettsäuren vorliegt:
Laurinsäure : Capronsäure = 50 : 0 bis 50 : 5 und/oder
Laurinsäure : Caprylsäure = 50 : 1 bis 50 : 20 und/oder
Laurinsäure : Pelargonsäure = 50 : 1 bis 50 : 20 und/oder
Laurinsäure : Caprinsäure = 50: 1 bis 50 : 20 und/oder
Laurinsäure : Myristinsäure = 50 : 5 bis 50 : 30 und/oder
Laurinsäure : Stearinsäure = 50 : 1 bis 50 : 50.

Besonders vorteilhaft ist, folgende Gewichtsverhältnisse zu wählen:
Laurinsäure : Capronsäure = 50 : 0 bis 50 : 1 und/oder
Laurinsäure : Caprylsäure = 50 : 2 bis 50 : 15 und/oder
Laurinsäure : Pelargonsäure = 50 : 2 bis 50 : 15 und/oder
Laurinsäure : Caprinsäure = 50 : 2 bis 50 : 15 und/oder
Laurinsäure : Myristinsäure = 50 : 10 bis 50 : 20 und/oder
Laurinsäure : Stearinsäure = 50 : 2 bis 50 : 20.

Wenn Laurinsäure im Gemisch mit mehreren weiteren Fettsäuren vorliegt, so ist es ferner günstig, ein Gemisch aus Laurinsäure, Caprinsäure und Caprylsäure zu verwenden, besonders, wenn das Gewichtsverhältnis gewählt wird aus dem Bereich
Laurinsäure : Caprylsäure : Caprinsäure = 50 : 1 : 1 bis 50 : 20 : 20.

Bevorzugt wird dann das Gewichtsverhältnis gewählt aus dem Bereich
Laurinsäure : Caprylsäure : Caprinsäure = 50 : 1 : 1 bis 50 : 5 : 5.

Die Verwendung von Capronsäure ist zwar erfindungsgemäß durchaus vorteilhaft und führt an sich zu Gemischen mit guter Wirkung gegen coryneforme Bakterien. Wegen des unangenehmen Eigengeruches der Capronsäure sollte deren Konzentration jedoch niedrig gehalten werden.

Die bevorzugte Ausführungsform der vorliegenden Erfindung besteht darin, das Verhältnis der Laurinsäure zu einer oder auch mehreren der anderen erfindungsgemäßen Fettsäuren so zu wählen, daß es dem Verhältnis in einem hydrierten (gehärteten) Schnitt von Fettsäuren aus natürlichem Kokosfettsäurengemisch entspricht.

Die Zusammensetzung der natürlichen Kokosfettsäuren beträgt etwa:
Laurinsäure : 44 - 51 Gew.-%
Myristinsäure : 13 - 18 Gew.-%
Palmitinsäure : 8 - 10 Gew.-%
Caprylsäure : 6 - 9 Gew.-%
Caprinsäure : 6 - 10 Gew.-%
Ölsäure : 5 - 8 Gew.-%
Stearinsäure : 1 - 3 Gew.-%
Linolsäure : 0 - 2 Gew.-%
Capronsäure : 0 - 1 Gew.-%

Die Zusammensetzung der gehärteten (hydrierten) Kokosfettsäuren beträgt etwa:
Laurinsäure : 44 - 51 Gew.-%
Myristinsäure : 13 - 18 Gew.-%
Palmitinsäure : 8 - 10 Gew.-%
Caprylsäure : 6 - 9 Gew.-%
Caprinsäure : 6 - 10 Gew.-%
Stearinsäure : 6 - 13 Gew.-%
Capronsäure : 0 - 1 Gew.-%

Laurinsäure (CAS-No. 143-07-7) ist von verschiedenen Anbietern erhältlich, beispielsweise von der Aarhus Oliefabrik A/S, von der Aceto Chemical Company Inc., von der Dansk Sojakagefabrik A/S, von Procter & Gamble Ltd. und anderen.

Hydrierte Kokosnußfettsäure ist unter der Handelsbezeichnung Hydrofol® Acid 631 von der Firma Ashland Chemical Company und unter der Bezeichnung Edenor® HK 8-18 von der Firma Henkel KGaA erhältlich.

Bevorzugt ist folgende Zusammensetzung der erfindungsgemäßen Gemische:
Laurinsäure : 1 - 99 Gew.-%
Myristinsäure : 0 - 18 Gew.-%
Palmitinsäure : 0 - 10 Gew.-%
Caprylsäure : 0 - 9 Gew.-%
Caprinsäure : 0 - 10 Gew.-%
Stearinsäure : 1 - 99 Gew.-%
Capronsäure : 0 - 1 Gew.-%
jeweils bezogen auf das Gesamtgewicht des Gemisches.

Besonders vorteilhafte Zusammensetzungen werden erhalten, wenn die Zusammensetzung des erfindungsgemäßen Gemisches wie folgt gewählt wird:
Laurinsäure : 10 - 90 Gew.-%
Myristinsäure : 13 - 18 Gew.-%
Palmitinsäure : 8 - 10 Gew.-%
Caprylsäure : 6 - 9 Gew.-%
Caprinsäure : 6 - 10 Gew.-%
Stearinsäure : 10 - 90 Gew.-%
Capronsäure : 0 - 1 Gew.-%
jeweils bezogen auf das Gesamtgewicht des Gemisches.

Insbesondere werden vorteilhafte Zusammensetzungen erhalten, wenn die Zusammensetzung des erfindungsgemäßen Gemisches wie folgt gewährt wird:
Laurinsäure : 44 - 51 Gew.-%
Myristinsäure : 13 - 18 Gew.-%
Palmitinsäure : 8 - 10 Gew.-%
Caprylsäure : 6 - 9 Gew.-%
Caprinsäure : 6 - 10 Gew.-%
Stearinsäure : 6 - 13 Gew.-%
Capronsäure : 0 - 1 Gew.-%,
jeweils bezogen auf das Gesamtgewicht des Gemisches.

Laut Spezifikation zeichnet sich Edenor® HK 8-18 durch folgenden ungefähren Gehalt an den erfindungsgemäßen Fettsäuren aus:
Laurinsäure : 48 Gew.-%
Myristinsäure : 18 Gew.-%
Palmitinsäure : 8 Gew.-%
Caprylsäure : 7 Gew.-%
Caprinsäure : 7 Gew.-%
Stearinsäure : 10 Gew.-%
Capronsäure : 1 Gew.-%
jeweils bezogen auf das Gesamtgewicht des Gemisches.

Die Verwendung dieses Handelsproduktes ist vorteilhaft.

In den Endformulierungen, also den desodorierenden Kosmetika, beträgt der Gehart an den erfindungsgemäßen Fettsäuregemischen vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Die erfindungsgemäßen kosmetischen Desodorantien können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren W/O- oder O/W-Emulsionen, z.B. Crèmes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Im allgemeinen sind desodorierende Gel-Stifte (Deo-Sticks) keine geeignete Darreichungsform für die erfindungsgemäßen Gemische, da sie zumeist hohe pH-Werte haben. Im basischen Milieu aber werden die erfindungsgemäßen Fettsäuregemische in die entsprechenden Seifen umgewandelt, welche nicht oder nicht genügend antimikrobiell wirksam sind.

Desodorierende Stifte, welche pH-Werte kleiner als 7 aufweisen, stellen dagegen durchaus vorteilhafte Darreichungsformen dar.

Als übliche kosmetische Trägerstoffe zur Herstellung der erfindungsgemäßen desodorierenden Mittel können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen kosmetischen Desodorantien, welche vorteilhaft als flüssige Zubereitungen mittels einer Roll-on-Vorrichtung auf die gewünschten Hautbereiche aufgetragen werden sollen, und die in den Mitteln in geringer Menge, z.B. 2 bis 5 Gewichts.-%, bezogen auf die Gesamt-Zusammensetzung, verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den desodorierenden kosmetischen Mitteln gemäß der Erfindung, deren pH-Wert vorzugsweise z.B. durch übliche Puffergemische auf 4,0 bis unter 7 insbesondere 5,0 bis 6,5, eingestellt wird, Parfüm, Farbstoffe, Antioxidantien (z.B. alpha-Tocopherol und seine Derivate oder Butylhydroxytoluol (BHT = 2,6-Di-Tert.-butyl-4-methylphenol) in Mengen von 0,01 bis 0,03 %, bezogen auf die Gesamt-Zusammensetzung), Suspendiermittel, Puffergemische oder andere übliche kosmetische Grundstoffe beigemischt werden.

Es ist für die vorliegende Erfindung wesentlich, daß der pH-Wert der erfindungsgemäßen kosmetischen Desodorantien kleiner als 7 ist. Jedenfalls ist für ein gegebenes Fettsäuregemisch im Einzelfalle durch einfaches Ausprobieren, ohne erfinderisches Dazutun, leicht zu ermitteln, welche exakte obere pH-Grenze zu beachten ist.

Die untere pH-Grenze wird allein von dermatologischen Gegebenheiten bestimmt. Da Kosmetika und Dermatika keinen pH-Wert kleiner als etwa 3,5 - 4 aufweisen sollten, kann dieser Bereich als untere Grenze angesehen werden. Dennoch sind die erfindungsgemäßen Zusammensetzungen grundsätzlich auch bei noch niedrigeren pH-Werten aktiv.

Vorteilhaft wird der pH-Wert der erfindungsgemäßen Formulierungen im sauren bis neutralen Bereich eingestellt, bevorzugt von 4,0 - unter 7, besonders bevorzugt von 5,0 - 6,5.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Zur Parfümierung sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der kosmetischen Mittel erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Sollen die erfindungsgemäßen Kombinationen in Pudersprays eingearbeitet werden, so können die Suspensionsgrundlagen dafür vorteilhaft gewählt werden aus der Gruppe

Kieselsäuregele (z.B. solche die unter dem Handelsnamen Aerosil® erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung.

### Beispiel 1

| Aerosolspray I | | |
|---|---|---|
| (a) Flüssige Phase | | Gew.-% |
| Octyldodecanol | | 0,50 |
| Kokosfettsäureschnitt | | 0,50 |
| (enthaltend | | |
| Capronsäure | 1 % | |
| Caprylsäure | 7 % | |
| Caprinsäure | 6 % | |
| Laurinsäure | 48 % | |
| Myristinsäure | 19 % | |
| Stearinsäure | 10 %) | |
| Parfüm | | q.s. |
| Ethylalkohol | | ad 100,00 |
| (b) Die unter (a) erhaltene flüssige Phase wird zusammen mit einem Propan/Butan 2,7 - Gemisch im Verhältnis 39 : 61 in Aerosolbehälter abgefüllt. | | |

### Beispiel 2

| Aerosolspray II | |
|---|---|
| (a) Flüssige Phase | Gew.-% |
| Octyldodecanol | 0,50 |
| Kokosfettsäureschnitt (Siehe Beispiel 1) | 0,20 |
| Parfüm | q.s. |
| Isopropylalkohol | ad 100,00 |
| (b) Die unter (a) erhaltene flüssige Phase wird zusammen mit einem Propan/Butan 2,7 - Gemisch im Verhältnis 39 : 61 in Aerosolbehälter abgefüllt. | |

### Beispiel 3

| Pumpspray I | |
|---|---|
| (a) | Gew.-% |
| Ethylalkohol | 60,00 |
| Glycerin | 1,00 |
| PEG-40-Hydriertes Ricinusöl | 2,00 |
| Kokosfettsäureschnitt (Siehe Beispiel 1) | 0,50 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 4

| Roll on - Gel I | Gew.-% |
|---|---|
| (a) | |
| 1,3-Butylenglycol | 2,00 |
| Hydroxyethylcellulose | 0,50 |
| (z.B. Tylose 4000, Hoechst) | |

| (b) | |
|---|---|
| Wasser | ad 100,00 |

| (c) | |
|---|---|
| Ethylalkohol | 60,00 |
| PEG-40-Hydriertes Ricinusöl | 2,00 |
| Kokosfettsäureschnitt (Siehe Beispiel 1) | 0,30 |
| Parfum | q.s. |

Die unter (a) genannten Bestandteile werden dispergiert, Wasser (b) wird zugegeben, bei Raumtemperatur quellen gelassen, eine Lösung der unter (c) genannten Bestandteile wird nach ca. 15 Minuten zugegeben. Die entstanden Mischung wird homogenisiert und kann abgefüllt werden.

### Beispiel 5

| Wachsstift 1 | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| Kokosfettsäureschnitt (Siehe Beispiel 1) | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 6

| Roll on - Emulsion I | |
|---|---|
| (a) | Gew.-% |
| Tricetearethphosphat | 0,30 |
| Octyldodecanol | 2,00 |
| C₁₂₋₁₅-Alkohol-Benzoate | 2,00 |
| Kokosfettsäureschnitt (Siehe Beispiel 1) | 0,50 |
| C₁₀₋₃₀-Alkylacrylate | 0,15 |

| (b) | |
|---|---|
| Wasser | ad 100,00 |
| NaOH | 0,05 |

| (c) | |
|---|---|
| Ethylalkohol | 10,00 |
| Parfum | q.s. |

Die unter (a) und (b) genannten Bestandteile werden jeweils unter Rühren auf 75° C erwärmt. Sodann werden die Bestandteile (a) zu (b) gegeben. Die Mischung wird auf 35° C abgekühlt. Aus den Bestandteilen (c) wird eine Lösung hergestellt, welche auf 35° C erwärmt wird und unter Rühren zur Mischung aus (a) und (b) gegeben wird.

### Beispiel 7

| Aerosolspray IV | |
|---|---|
| (a) Flüssige Phase | Gew.-% |
| Octyldodecanol | 0,50 |
| Fettsäuregemisch (Siehe Beispiel 7) | 0,20 |
| Parfüm | q.s. |
| Isopropylalkohol | ad 100,00 |
| (b) Die unter (a) erhaltene flüssige Phase wird zusammen mit einem Propan/Butan 2,7 - Gemisch im Verhältnis 39 : 61 in Aerosolbehälter abgefüllt. | |

### Beispiel 8

| Pumpspray II | |
|---|---|
| (a) | Gew.-% |
| Ethylalkohol | 60,00 |
| Glycerin | 1,00 |
| PEG-40-Hydriertes Ricinusöl | 2,00 |
| Fettsäuregemisch (Siehe Beispiel 7) | 0,50 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 9

| Roll on - Gel II | Gew.-% |
|---|---|
| (a) | |
| 1,3-Butylenglycol | 2,00 |
| Hydroxyethylcellulose (z.B. Tylose 4000, Hoechst) | 0,50 |

| (b) | |
|---|---|
| Wasser | ad 100,00 |

| (c) | |
|---|---|
| Ethylalkohol | 60,00 |
| PEG-40-Hydriertes Ricinusöl | 2,00 |
| Fettsäureschnitt (siehe Beispiel 7) | 0,30 |
| Parfum | q.s. |

Die unter (a) genannten Bestandteile werden dispergiert, Wasser (b) wird zugegeben, bei Raumtemperatur quellen gelassen, eine Lösung der unter (c) genannten Bestandteile wird nach ca. 15 Minuten zugegeben. Die entstanden Mischung wird homogenisiert und kann abgefüllt werden.

### Beispiel 10

| Wachsstift II | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| Fettsäuregemisch (siehe Beispiel 7) | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 11

| Roll on - Emulsion II | |
|---|---|
| (a) | Gew.-% |
| Tricetearethphosphat | 0,30 |
| Octyldodecanol | 2,00 |
| C₁₂₋₁₅-Alkohol-Benzoate | 2,00 |
| Fettsäuregemisch (siehe Beispiel 7) | 0,50 |
| C₁₀₋₃₀-Alkylacrylate | 0,15 |

| (b) | |
|---|---|
| Wasser | ad 100,00 |
| NaOH | 0,05 |

| (c) | |
|---|---|
| Ethylalkohol | 10,00 |
| Parfum | q.s. |

Die unter (a) und (b) genannten Bestandteile werden jeweils unter Rühren auf 75° C erwärmt. Sodann werden die Bestandteile (a) zu (b) gegeben. Die Mischung wird auf 35° C abgekühlt. Aus den Bestandteilen (c) wird eine Lösung hergestellt, welche auf 35° C erwärmt wird und unter Rühren zur Mischung aus (a) und (b) gegeben wird.

### Beispiel 12

| Pumpspray III | |
|---|---|
| (a) | Gew.-% |
| Ethylalkohol | 60,00 |
| Glycerin | 1,00 |
| PEG-40-Hydriertes Ricinusöl | 2,00 |
| Fettsäuregemisch (siehe Beispiel 7) | 0,50 |
| Parfum | q.s. |

| (b) | |
|---|---|
| Wasser | auf 100,00 |

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 13

| Roll on - Gel III | Gew.-% |
|---|---|
| (a) | |
| 1,3-Butylenglycol | 2,00 |
| Hydroxyethylcellulose (z.B. Tylose 4000, Hoechst) | 0,50 |

| (b) | |
|---|---|
| Wasser | ad 100,00 |

| (c) | |
|---|---|
| Ethylalkohol | 60,00 |
| PEG-40-Hydriertes Ricinusöl | 2,00 |
| Fettsäureschnitt (siehe Beispiel 7) | 0,30 |
| Parfum | q.s. |

Die unter (a) genannten Bestandteile werden dispergiert, Wasser (b) wird zugegeben, bei Raumtemperatur quellen gelassen, eine Lösung der unter (c) genannten Bestandteile wird nach ca. 15 Minuten zugegeben. Die entstanden Mischung wird homogenisiert und kann abgefüllt werden.

### Beispiel 14

| Wachsstift III | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| Fettsäuregemisch (siehe Beispiel 7) | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 15

| Roll on - Emulsion III | |
|---|---|
| (a) | Gew.-% |
| Tricetearethphosphat | 0,30 |
| Octyldodecanol | 2,00 |
| C₁₂₋₁₅-Alkohol-Benzoate | 2,00 |
| Fettsäuregemisch (siehe Beispiel 7) | 0,50 |
| C₁₀₋₃₀-Alkylacrylate | 0,15 |

| (b) | |
|---|---|
| Wasser | ad 100,00 |
| NaOH | 0,05 |

| (c) | |
|---|---|
| Ethylalkohol | 10,00 |
| Parfum | q.s. |

Die unter (a) und (b) genannten Bestandteile werden jeweils unter Rühren auf 75° C erwärmt. Sodann werden die Bestandteile (a) zu (b) gegeben. Die Mischung wird auf 35° C abgekühlt. Aus den Bestandteilen (c) wird eine Lösung hergestellt, welche auf 35° C erwärmt wird und unter Rühren zur Mischung aus (a) und (b) gegeben wird.

Anhand der folgenden Versuche soll die synergistische Wirkung der erfindungsgemäßen Gemische verdeutlicht werden:

### Versuch 1:

### Eingesetzte Lösungen:

| | | |
|---|---|---|
| AC-Medium: | 37 g Brain Heart Infusion, 5 g Glucose, 1 ml Tween 80 ad 1 l H₂O | |
| Enthemmungsmedium: | (AC-Medium + 3 % Tween 80 + 0,3 % Lecithin + 0,1 % Histidin) | |
| Testsubstanz 1: | Laurinsäure (0,2 %) | |
| Testsubstanz 2: | Kokosfettsäureschnitt (0,4 %), enthaltend | |
| | Capronsäure | 1 % |
| | Caprylsäure | 7 % |
| | Caprinsäure | 6 % |
| | Laurinsäure | 48 % |
| | Myristinsäure | 19 % |
| | Stearinsäure | 10 % |
| Testsubstanz 3: | Stearinsäure (0,04 %), entsprechend dem Stearinsäuregehalt in Tstsubstanz 2 | |
| Kontrolle: | AC-Medium ohne Wirkstoff | |
| Testlösungen: | Jeweilige Testsubstanz in AC-Medium | |

### a) Suspensionstest

60 ml AC-Medium wurden mit 0,4 ml einer frischen Übemachtkultur des Testorganismus Corynebacterium xerosis (C. xerosis) beimpft. Das Kulturgefäß wurde bei 30° C mit 250 Upm geschüttelt, bis eine Zelldichte von 10⁵ bis 10⁶ Zellen/ml erreicht war. Die Testsubstanzen wurden eingewogen und jeweils in 2,5 ml AC-Medium unter kurzem Erwärmen auf 60° C gelöst. Anschließend wurden je 2,5 ml Bakteriensuspension mit den so gewonnenen Testlösungen versetzt und unter Beibehaltung der Wachstumsbedingungen 2 Std. inkubiert.Sodann wurden 100 µl der inkubierten Testlösungen zu 900 µl Enthemmungsmedium gegeben und der Bakterientiter durch Ausplattieren auf Nährmedium bestimmt.

### Ergebnis:

Die mit Testsubstanz 1 versetzte Kultur wies nach Abschluß des Experiments 4 * 10² Zellen/ml auf.

Die mit Testsubstanz 2 versetzte Kultur wies nach Abschluß des Experiments 2 * 10² Zellen/ml auf.

Die mit Testsubstanz 3 versetzte Kultur wies nach Abschluß des Experiments 5 * 10² Zellen/ml auf.

Die Kontrolle wies nach Abschluß des Experiments 5 * 10⁵ Zellen/ml auf.

### b) Wachstumskurve

50 ml AC-Medium wurden mit 0,2 ml einer frischen Übemachtkultur des Testorganismus C.xerosis beimpft. Das Kulturgefäß wurde bei 30° C mit 250 Upm geschüttelt, bis eine Zelldichte von 10⁵ bis 10⁶ Zellen/ml erreicht war. Die Testsubstanzen wurden eingewogen und jeweils in 5 ml AC-Medium unter kurzem Erwärmen auf 60° C gelöst. Anschließend wurden 5 ml-Aliquots der Bakterienkultur in 100 ml fassende Erlenmeyerkolben gegeben und mit je 5 ml Testlösung versetzt. Nach sorgfältigem Durchmischen wurden unter Beibehaltung der Wachstumsbedingungen zu verschiedenen Zeitpunkten Proben entnommen und deren Bakterientiter durch Ausplattieren von Verdünnungen auf Nährmedien bestimmt.

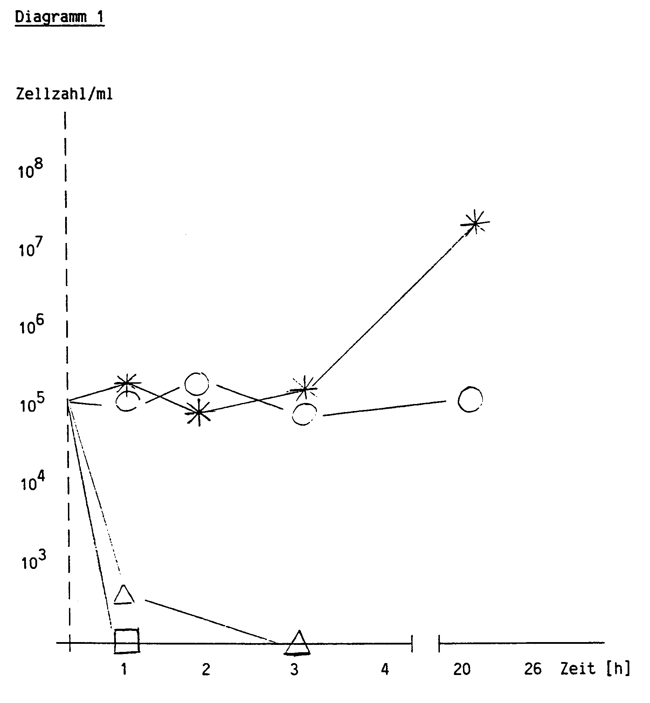

In Diagramm 1 sind die Wachstumskurven für die vorabbeschriebenen Versuchsansätze aufgezeigt. Es bedeuten
-△- : Testlösung 1 (0,2 % Laurinsäure in AC-Medium)
-□- : Testlösung 2 (0,4 % Kokossäureschnitt in AC-Medium)
-○ - : Testlösung 3 (0,04 % Stearinsäure in AC-Medium)
- 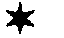 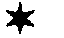 - : Kontrolle (Wirkstoff-freies AC-Medium)

Wie aus dem Diagramm ersichtlich ist, sind die erfindungsgemäßen synergistischen Mischungen den Einzelsubstanzen deutlich überlegen.

### Versuch 2

### Eingesetzte Lösungen:

- AC-Medium und Enthemmungsmedium:: Wie in Versuch 1
- Testsubstanz 5:: Glycerinmonolaurat
- Testsubstanz 6:: Kokosfettsäureschnitt (Zusammensetzung wie in Versuch 1).
- Testsubstanz 7:: Gemisch aus Glycerinmonolaurat und Kokosfettsäureschnitt (Zusammensetzung wie in Versuch 1).
- Kontrolle:: Wirkstoff-freies AC-Medium
- Testlösungen:: Jeweilige Testsubstanz in AC-Medium

### Ergebnis:

### Wachstumskurve

50 ml AC-Medium wurden mit 0,2 ml einer frischen Übernachtkultur des Testorganismus C.xerosis beimpft. Das Kulturgefäß wurde bei 30° C mit 250 Upm geschüttelt, bis eine Zelldichte von 10⁵ bis 10⁶ Zellen/ml erreicht war. Die Testsubstanzen wurden eingewogen und jeweils in 5 ml AC-Medium unter kurzem Erwärmen auf 60° C gelöst. Anschließend wurden 5 ml-Aliquots der Bakterienkultur in 100 ml fassende Erlenmeyerkolben gegeben und mit je 5 ml Testlösung versetzt. Nach sorgfältigem Durchmischen wurden unter Beibehaltung der Wachstumsbedingungen zu verschiedenen Zeitpunkten Proben entnommen und deren Bakterientiter durch Ausplattieren von Verdünnungen auf Nährmedien bestimmt.

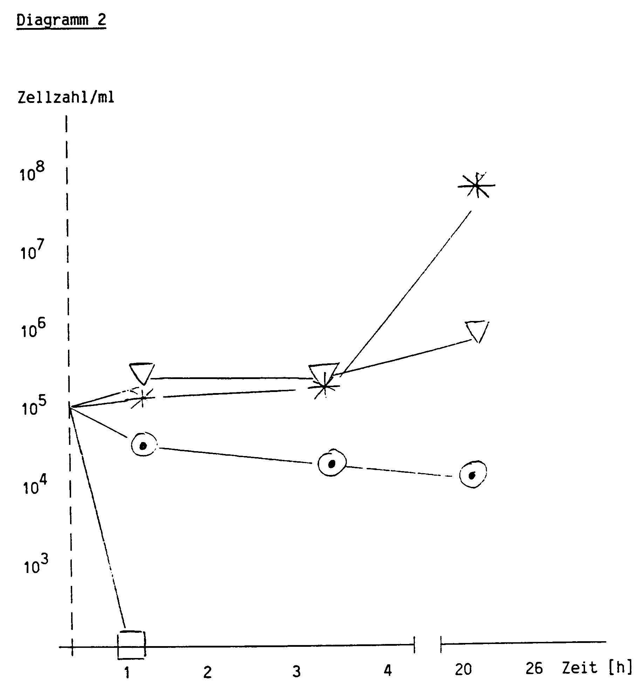

In Diagramm 2 sind die Wachstumskurven für die vorabbeschriebenen Versuchsansätze aufgezeigt. Es bedeuten
-∇ -: Testlösung 5 (0,5 % Glycerinmonolaurat in AC-Medium)
-□ -: Testlösung 6 (0,4 % Kokosfettsäureschnitt in AC-Medium)
-·⃝ -: Testlösung 7 (0,5 % Glycerinmonolaurat und 0,4 % Kokosfettsäureschnitt in AC-Medium)
- 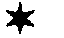 -: Kontrolle

Wie aus Diagramm 2 ersichtlich ist, wird die antimikrobielle Wirksamkeit der erfindungsgemäßen Fettsäuregemische durch Zusatz von Glycerinmonolaurat verringert. Wo das reine Fettsäuregemisch bereits nach einer Stunde die Zellzahl unter 100/ml senkt, sinkt die Zellzahl bei einem Zusatz von 0,5 % Glycerinmonolaurat zum Fettsäuregemisch über den Versuchszeitraum von 20 Stunden praktisch nicht ab.

### Versuch 3

### Eingesetzte Lösungen:

| | |
|---|---|
| AC-Medium und Enthemmungsmedium: | Wie in Versuch 1 |
| Testsubstanz 8: | Kokosfettsäureschnitt (Zusammensetzung wie in Versuch 1) |
| Testlösung 8: | Testsubstanz 8 (0,4 % in AC-Medium), Puffer. pH: 4,0 |
| Testlösung 9: | wie 8, pH: 5,0 |
| Testsubstanz 10: | wie 8, pH: 6,0 |
| Testsubstanz 11: | wie 8, pH: 7,0 |
| Testsubstanz 12: | wie 8, pH: 8,0 |
| Kontrolle: | Wirkstoff-freies AC-Medium, gepuffert auf den pH-Wert der jeweiligen Testlösung |

60 ml AC-Medium wurden mit 0,4 ml einer frischen Übernachtkultur des Testorganismus C. xerosis beimpft. Das Kulturgefäß wurde bei 30° C mit 250 Upm geschüttelt, bis eine Zelldichte von 10⁵ bis 10⁶ Zellen/ml erreicht war.

Die Testsubstanzen wurden eingewogen und jeweils in 2,5 ml eines 50 mM Tris-Puffers mit dem gewünschten pH-Wert (Bereich 4,0 - 8,0) gelöst.

Anschließend wurden 2,5 ml der Bakteriensuspensionen mit den so gewonnenen Lösungen der Testsubstanzen in Puffer versetzt und unter Beibehaltung der Wachstumsbedingungen 2 Std. bei 30° C inkubiert.

Sodann wurden 100 µl der inkubierten Testlösungen zu 900 µl Enthemmungsmedium gegeben und der Bakterientiter durch Ausplattieren von Verdünnungen auf Nährmedium bestimmt.

### Ergebnis:

Testlösung 8 wies nach Abschluß des Experiments weniger als 100 Zellen/ml auf. Die Kontrolle dazu wies nach Abschluß des Experiments ca. 10⁵ Zellen/ml auf.

Testlösung 9 wies nach Abschluß des Experiments weniger als 100 Zellen/ml auf. Die Kontrolle dazu wies nach Abschluß des Experiments ca. 10⁴ Zellen/ml auf.

Testlösung 10 wies nach Abschluß des Experiments weniger als 100 Zellen/ml auf. Die Kontrolle dazu wies nach Abschluß des Experiments ca. 10⁵ Zellen/ml auf.

Testlösung 11 wies nach Abschluß des Experiments weniger als 100 Zellen/ml auf. Die Kontrolle dazu wies nach Abschluß des Experiments ca. 5 * 10³ Zellen/ml auf.

Testlösung 12 wies nach Abschluß des Experiments ca. 10⁵ Zellen/ml auf. Die Kontrolle dazu wies nach Abschluß des Experiments ca. 10⁵ Zellen/ml auf.

Wie Versuch 3 zeigt, sind Formulierungen des erfindungsgemäßen Fettsäuregemisches inaktiv, wenn sie in einem pH-Bereich größer 8 gepuffert sind. Die Grenze der Aktivität liegt zwischen pH 7 und pH 8. Wo die erfindungsgemäßen Gemische beim Neutralpunkt noch voll aktiv sind, ist bei pH 8 keine nennenswerte Aktivität festzustellen: In der Testlösung 12 und der dazugehörigen Kontrolle wurden praktisch identische Bakterienkonzentrationen nachgewiesen.

## Patentansprüche

1. Kosmetische Desodorantien mit einem wirksamen Gehalt eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C₆₋₂₀
(c) unter Verzicht auf Glycerylfettsäureester, ethoxylierte Glycerylfettsäureester und propoxylierte Glycerylfettsäureester
(d) im pH-Bereich unter 7 vorliegend.

2. Kosmetische Desodorantien nach Anspruch 1, dadurch gekennzeichnet, daß die weiteren gesättigte unverzweigte Fettsäuren gewählt werden aus der Gruppe Capronsäure (Hexansäure), Caprylsäure (Octansäure), Pelargonsäure (Nonansäure), Caprinsäure (Decansäure), Myristinsäure (Tetradecansäure), Palmitinsäure (Hexadecansäure) und Stearinsäure (Octadecansäure).

3. Kosmetische Desodorantien nach Anspruch 1, dadurch gekennzeichnet, daß folgende Gewichtsverhältnisse der Fettsäuren untereinander vorliegen:
Laurinsäure : Capronsäure = 50 : 0 bis 50 : 5 und/oder
Laurinsäure : Caprylsäure = 50 : 1 bis 50 : 20 und/oder
Laurinsäure : Pelargonsäure = 50 : 1 bis 50 : 20 und/oder
Laurinsäure : Caprinsäure = 50 : 1 bis 50 : 20 und/oder
Laurinsäure : Myristinsäure = 50 : 5 bis 50 : 30 und/oder
Laurinsäure : Stearinsäure = 50 : 1 bis 50 : 50,
bevorzugt:
Laurinsäure : Capronsäure = 50 : 0 bis 50 : 1 und/oder
Laurinsäure : Caprylsäure = 50 : 2 bis 50 : 15 und/oder
Laurinsäure : Pelargonsäure = 50 : 2 bis 50 : 15 und/oder
Laurinsäure : Caprinsäure = 50 : 2 bis 50 : 15 und/oder
Laurinsäure : Myristinsäure = 50 : 10 bis 50 : 20 und/oder
Laurinsäure : Stearinsäure = 50 : 2 bis 50 : 20.

4. Kosmetische Desodorantien nach Anspruch 1, dadurch gekennzeichnet, daß folgende Gewichtsverhältnisse der Fettsäuren untereinander vorliegen:
Laurinsäure : Caprylsäure : Caprinsäure = 50 : 1 : 1 bis 50 : 20 : 20,
bevorzugt:
Laurinsäure : Caprylsäure : Caprinsäure = 50 : 1 : 1 bis 50 : 5 : 5.

5. Kosmetische Desodorantien nach Anspruch 1, dadurch gekennzeichnet, daß die erfindungsgemäßen Gemische sich
zusammensetzen wie folgt:
Laurinsäure : 1 - 99 Gew.-%
Myristinsäure : 0-18 Gew.-%
Palmitinsäure : 0 - 10 Gew.-%
Caprylsäure : 0 - 9 Gew.-%
Caprinsäure : 0 - 10 Gew.-%
Stearinsäure : 1 - 99 Gew.-%
Capronsäure : 0 - 1 Gew.-%,
bevorzugt
Laurinsäure : 10 - 90 Gew.-%
Myristinsäure : 13 - 18 Gew.-%
Palmitinsäure : 8 - 10 Gew.-%
Caprylsäure : 6 - 9 Gew.-%
Caprinsäure : 6 - 10 Gew.-%
Stearinsäure : 10 - 90 Gew.-%
Capronsäure : 0 - 1 Gew.-%,
besonders bevorzugt
Laurinsäure : 44 - 51 Gew.-%
Myristinsäure : 13 - 18 Gew.-%
Palmitinsäure : 8 - 10 Gew.-%
Caprylsäure : 6 - 9 Gew.-%
Caprinsäure : 6 - 10 Gew.-%
Stearinsäure : 6 - 13 Gew.-%
Capronsäure : 0 - 1 Gew.-%,
jeweils bezogen auf das Gesamtgewicht des Gemisches.

6. Kosmetische Desodorantien nach Anspruch 1, enthaltend Gemische, in welchen das Verhältnis der Fettsäuren zueinander demjenigen entspricht, wie es in einem hydrierten (gehärteten) Schnitt von Fettsäuren aus dem natürlichen Kokosfettsäurengemisch auftritt.

7. Kosmetische Desodorantien nach Anspruch 1, enthaltend
(a) Laurinsäure und weitere gesättigte unverzweigte Fettsäuren, die in ihrer Zusammensetzung einem hydrierten (gehärteten) Schnitt von Fettsäuren aus dem natürlichen Kokosfettsäurengemisch entsprechen oder
(b) einen solchen Schnitt selbst.

8. Kosmetische Desodorantien nach Anspruch 1, dadurch gekennzeichnet daß sie in Form von Aerosolen, als Roll-on, als W/O- oder O/W-Emulsionen, z.B. Crèmes oder Lotionen, als desodorierende Tinkturen, desodorierende Intimreinigungsmittel, desodorierende Shampoos, desodorierende Dusch- oder Badezubereitungen, desodorierende Puder oder desodorierende Pudersprays vorliegen.

9. Kosmetische Desodorantien nach Anspruch 1, dadurch gekennzeichnet, daß sie in Form von desodorierenden Stiften, die einen pH-Wert unter 7 aufweisen, vorliegen.

10. Verwendung eines Gemisches aus
Laurinsäure : 1 - 99 Gew.-%
Myristinsäure : 0 - 18 Gew.-%
Palmitinsäure : 0 - 10 Gew.-%
Caprylsäure : 0 - 9 Gew.-%
Caprinsäure : 0 - 10 Gew.-%
Stearinsäure : 1 - 99 Gew.-%
Capronsäure : 0 - 1 Gew.-%,
bevorzugt
Laurinsäure : 10 - 90 Gew.-%
Myristinsäure : 13 - 18 Gew.-%
Palmitinsäure : 8 - 10 Gew.-%
Caprylsäure : 6 - 9 Gew.-%
Caprinsäure : 6 - 10 Gew.-%
Stearinsäure : 10 - 90 Gew.-%
Capronsäure : 0 - 1 Gew.-%,
besonders bevorzugt
Laurinsäure : 44 - 51 Gew.-%
Myristinsäure : 13 - 18 Gew.-%
Palmitinsäure : 8 - 10 Gew.-%
Caprylsäure : 6 - 9 Gew.-%
Caprinsäure : 6 - 10 Gew.-%
Stearinsäure : 6 - 13 Gew.-%
Capronsäure : 0 - 1 Gew.-%,
bezogen auf das Gesamtgewicht des Gemisches, als wirksames Prinzip in kosmetischen Desodorantien.

## Claims

1. Cosmetic deodorants having an active content of a mixture of
(a) dodecanoic acid (lauric acid)
(b) at least one other fatty acid chosen from the group consisting of unbranched saturated fatty acids having a chain length of C₆-C₂₀
(c) with omission of glyceryl fatty acid esters, ethoxylated glyceryl fatty acid esters and propoxylated glyceryl fatty acid esters
(d) in the pH range below 7.

2. Cosmetic deodorants according to Claim 1, characterized in that the other saturated unbranched fatty acids are chosen from the group consisting of caproic acid (hexanoic acid), caprylic acid (octanoic acid), pelargonic acid (nonanoic acid), capric acid (decanoic acid), myristic acid (tetradecanoic acid), palmitic acid (hexadecanoic acid) and stearic acid (octadecanoic acid).

3. Cosmetic deodorants according to Claim 1, characterized in that the following weight ratios of the fatty acids with respect to one another are present:
Lauric acid: caproic acid = 50 : 0 to 50 : 5 and/or
Lauric acid: caprylic acid = 50 : 1 to 50 : 20 and/or
Lauric acid: pelargonic acid = 50 : 1 to 50 : 20 and/or
Lauric acid: capric acid = 50 : 1 to 50 : 20 and/or
Lauric acid: myristic acid = 50 : 5 to 50 : 30 and/or
Lauric acid: stearic acid = 50 : 1 to 50 : 50,
preferably:
Lauric acid: caproic acid = 50 : 0 to 50 : 1 and/or
Lauric acid: caprylic acid = 50 : 2 to 50 : 15 and/or
Lauric acid: pelargonic acid = 50 : 2 to 50 : 15 and/or
Lauric acid: capric acid = 50 : 2 to 50 : 15 and/or
Lauric acid: myristic acid = 50 : 10 to 50 : 20 and/or
Lauric acid: stearic acid = 50 : 2 to 50 : 20.

4. Cosmetic deodorants according to Claim 1, characterized in that the following weight ratios of the fatty acids with respect to one another are present:
Lauric acid : caprylic acid : capric acid = 50 : 1 : 1 to 50 : 20 : 20,
preferably:
Lauric acid : caprylic acid : capric acid = 50 : 1 : 1 to 50 : 5 : 5.

5. Cosmetic deodorants according to Claim 1, characterized in that the mixtures according to the invention are composed as follows:
Lauric acid : 1 - 99 % by weight
Myristic acid : 0 - 18 % by weight
Palmitic acid : 0 - 10 % by weight
Caprylic acid : 0 - 9 % by weight
Capric acid : 0 - 10 % by weight
Stearic acid : 1 - 99 % by weight
Caproic acid : 0 - 1 % by weight,
preferably
Lauric acid : 10 - 90 % by weight
Myristic acid : 13 - 18 % by weight
Palmitic acid : 8 - 10 % by weight
Caprylic acid : 6 - 9 % by weight
Capric acid : 6 - 10 % by weight
Stearic acid : 10 - 90 % by weight
Caproic acid : 0 - 1 % by weight,
particularly preferably
Lauric acid : 44 - 51 % by weight
Myristic acid : 13 - 18 % by weight
Palmitic acid : 8 - 10 % by weight
Caprylic acid : 6 - 9 % by weight
Capric acid : 6 - 10 % by weight
Stearic acid : 6 - 13 % by weight
Caproic acid : 0 - 1 % by weight,
in each case based on the total weight of the mixture.

6. Cosmetic deodorants according to Claim 1, comprising mixtures in which the ratio of the fatty acids with respect to one another corresponds to that which occurs in a hydrogenated (hardened) cut of fatty acids from the naturally occurring coconut fatty acid mixture.

7. Cosmetic deodorants according to Claim 1, comprising
(a) lauric acid and other saturated unbranched fatty acids which correspond in their composition to a hydrogenated (hardened) cut of fatty acids from the naturally occurring coconut fatty acid mixture or
(b) such a cut itself.

8. Cosmetic deodorants according to Claim 1, characterized in that they are in the form of aerosols, in the form of a roll-on, in the form of water-in-oil or oil-in-water emulsions, for example creams or lotions, or in the form of deodorizing tinctures, deodorizing intimate cleansing agents, deodorizing shampoos, deodorizing shower or bath formulations, deodorizing powders or deodorizing powder sprays.

9. Cosmetic deodorants according to Claim 1, characterized in that they are in the form of deodorizing sticks which have a pH below 7.

10. Use of a mixture of
Lauric acid : 1 - 99 % by weight
Myristic acid : 0 - 18 % by weight
Palmitic acid : 0 - 10 % by weight
Caprylic acid : 0 - 9 % by weight
Capric acid : 0 - 10 % by weight
Stearic acid : 1 - 99 % by weight
Caproic acid : 0 - 1 % by weight,
preferably
Lauric acid : 10 - 90 % by weight
Myristic acid : 13 - 18 % by weight
Palmitic acid : 8 - 10 % by weight
Caprylic acid : 6 - 9 % by weight
Capric acid : 6 - 10 % by weight
Stearic acid : 10 - 90 % by weight
Caproic acid : 0 - 1 % by weight,
particularly preferably
Lauric acid : 44 - 51 % by weight
Myristic acid : 13 - 18 % by weight
Palmitic acid : 8 - 10 % by weight
Caprylic acid : 6 - 9 % by weight
Capric acid : 6 - 10 % by weight
Stearic acid : 6 - 13 % by weight
Caproic acid : 0 - 1 % by weight,
based on the total weight of the mixture, as the active principle in cosmetic deodorants.

## Revendications

1. Agents cosmétiques déodorants ayant une teneur efficace d'un mélange
(a) d'acide dodécanoïque (acide laurique),
(b) d'au moins un acide gras supplémentaire, choisi parmi le groupe des acides gras saturés non ramifiés d'une longueur de chaîne de C₆₋₂₀,
(c) à l'exception d'esters d'acides gras glycériques, d'esters d'acides gras glycériques éthoxylés et d'esters d'acides gras glycériques propoxylés,
(d) présents dans un domaine de pH inférieur à 7.

2. Agents cosmétiques déodorants selon la revendication 1, caractérisés en ce que les acides gras supplémentaires saturés non ramifiés sont choisis parmi le groupe de l'acide caproïque (acide hexanoïque), de l'acide caprylique (acide octanoïque), l' acide pélargonique (acide nonanoïque), de l'acide caprique (acide décanoïque), de l'acide myristique (acide tétradécanoïque), de l'acide palmitique (acide hexadécanoïque) et de l'acide stéarique (acide octadécanoïque).

3. Agents cosmétiques déodorants selon la revendication 1, caractérisés en ce que sont présentes les proportions suivantes en poids réciproques des acides gras :
Acide laurique : acide caproïque = 50 : 0 à 50 : 5 et/ou
Acide laurique : acide caprylique = 50 : 1 à 50 : 20 et/ou
Acide laurique : acide pélargonique = 50 : 1 à 50 : 20 et/ou
Acide laurique : acide caprique = 50 : 1 à 50 : 20 et/ou
Acide laurique : acide myristique = 50 : 5 à 50 : 30 et/ou
Acide laurique : acide stéarique = 50 : 1 à 50 : 50
de préférence :
Acide laurique : acide caproïque = 50 : 0 à 50 : 1 et/ou
Acide laurique : acide caprylique = 50 : 2 à 50 : 15 et/ou
Acide laurique : acide pélargonique = 50 : 2 à 50 : 15 et/ou
Acide laurique : acide caprique = 50 : 2 à 50 : 15 et/ou
Acide laurique : acide myristique = 50 : 10 à 50 : 20 et/ou
Acide laurique : acide stéarique = 50 : 2 à 50 : 20.

4. Agents cosmétiques déodorants selon la revendication 1, caractérisés en ce que sont présentes les proportions suivantes en poids réciproques des acides gras :
Acide laurique : acide caprylique : acide caprique = 50 : 1 : 1 à 50 : 20 : 20,
de préférence:
acide laurique : acide caprylique : acide caprique = 50 : 1 : 1 à 50 : 5 : 5.

5. Agents cosmétiques déodorants selon la revendication 1, caractérisés en ce que les mélanges selon l'invention se composent comme suit :
Acide laurique : 1 - 99 % en poids
Acide myristique : 0 - 18 % en poids
Acide palmitique : 0 - 10 % en poids
Acide caprylique : 0 - 9 % en poids
Acide caprique : 0 - 10 % en poids
Acide stéarique : 1 - 99 % en poids
Acide caproïque : 0 - 1 % en poids
de préférence
Acide laurique : 10 - 90 % en poids
Acide myristique : 13 - 18 % en poids
Acide palmitique : 8 - 10 % en poids
Acide caprylique : 6 - 9 % en poids
Acide caprique : 6 - 10 % en poids
Acide stéarique : 10 - 90 % en poids
Acide caproïque : 0 - 1 % en poids.
en particulier de préférence
Acide laurique : 44 - 51 % en poids
Acide myristique : 13 - 18 % en poids
Acide palmitique : 8 - 10 % en poids
Acide caprylique : 6 - 9 % en poids
Acide caprique : 6 - 10 % en poids
Acide stéarique : 6 - 13 % en poids
Acide caproïque : 0 - 1 % en poids,
à chaque fois par rapport au poids total du mélange.

6. Agents cosmétiques déodorants selon la revendication 1, comprenant des mélanges, dans lesquels le rapport des acides gras les uns par rapport aux autres correspond à celui qui se produit dans une coupe hydrogénée (durcie) d'acides gras en provenance du mélange naturel d'acides gras d'huile de coco.

7. Agents cosmétiques déodorants selon la revendication 1, comprenant
(a) l'acide laurique et des acides gras non ramifiés saturés supplémentaires, qui correspondent dans leur composition à une coupe hydrogénée (durcie) d'acides gras en provenance du mélange naturel d'acides gras d'huile de coco ou
(b) une coupe de ce genre proprement dite.

8. Agents cosmétiques déodorants selon la revendication 1, caractérisés en ce qu'ils se présentent sous la forme d'aérosols, de produits Roll-on, en tant qu'émulsions eau/solvant organique ou solvant organique/eau, par exemple des crèmes ou des lotions, en tant qu'extraits alcooliques déodorants, en tant qu'agents déodorants d'hygiène intime, en tant que shampooings déodorants, en tant que préparations déodorantes pour la douche ou le bain, en tant que poudres déodorantes ou en tant que poudres-aérosol déodorantes.

9. Agents déodorants selon la revendication 1, caractérisés en ce qu'ils se présentent sous la forme de bâtons déodorants, qui présentent une valeur de pH inférieure à 7.

10. Utilisation d'un mélange de
Acide laurique : 1 - 99 % en poids
Acide myristique : 0 - 18 % en poids
Acide palmitique : 0 - 10 % en poids
Acide caprylique : 0 - 9 % en poids
Acide caprique : 0 - 10 % en poids
Acide stéarique : 1 - 99 % en poids
Acide caproïque : 0 - 1 % en poids
de préférence
Acide laurique : 10 - 90 % en poids
Acide myristique : 13 - 18 % en poids
Acide palmitique : 8 - 10 % en poids
Acide caprylique : 6 - 9 % en poids
Acide caprique : 6 - 10 % en poids
Acide stéarique : 10 - 90 % en poids
Acide caproïque : 0 - 1 % en poids.
en particulier de préférence
Acide laurique : 44 - 51 % en poids
Acide myristique : 13 - 18 % en poids
Acide palmitique : 8 - 10 % en poids
Acide caprylique : 6 - 9 % en poids
Acide caprique : 6 - 10 % en poids
Acide stéarique : 6 - 13 % en poids
Acide caproïque : 0 - 1 % en poids,
par rapport au poids total du mélange, en tant que principe actif dans des agents cosmétiques déodorants.
